# EUROPEAN PATENT APPLICATION

(11) **EP 4 410 320 A1**
(43) Date of publication of application: **07.08.2024**
(21) Application number: 24156030.9
(22) Date of filing: 06.02.2024
(51) Int. Cl.: A61L 15/14, A61L 15/64, A61L 26/00, A61L 27/38, A61L 27/60, B33Y 10/00

(54) **REUSE OF FIBROBLASTS FOR SKIN AUGMENTATION**

(30) Priority: 06.02.2023 US 202363483349 P; 16.03.2023 US 202363490542 P
(71) Applicant: FibroBiologics, Inc., Houston, Texas 77289 (US)
(72) Inventor: O'HEERON, Pete, Houston, 77059 (US); KHOJA, Hamid, Houston, 77289 (US)
(74) Representative: J A Kemp LLP

(57) **Abstract**

Embodiments of the disclosure include compositions and methods for providing an effective amount of activated or unactivated single-cell or spheroid fibroblasts, exosomes from fibroblasts, lysate from fibroblasts, apoptotic bodies from fibroblasts, and/or fibroblast-related products that are comprised in and/or on a substrate, such as a synthetic polymer or biopolymer scaffolding or biologic mesh, thereby generating a material to cover at least one wound and/or at least one burn of an individual.

## Description

### CLAIM OF PRIORITY

This application claims the benefit of U.S. Provisional Patent Application No. 63/483,349, filed on February 6, 2023, and U.S. Provisional Patent Application No. 63/490,542, filed on March 16, 2023, both of which are incorporated by reference in full herein.

### TECHNICAL FIELD

Embodiments of the disclosure concern at least the fields of cell biology, molecular biology, immunology, and medicine.

### BACKGROUND

Fibroblasts are no longer considered mere structural components of organs but as dynamic participants in multiple systems, such as the immune and the complex interplay of multiple systems involved in wound healing.

Skin is the largest organ in the human body encompassing about 15% of the total body weight, and serves multiple complex functions such as protection from external physical, chemical, and microbial impacts, maintenance of temperature and electrolyte balance, serves as a biofactory for the synthesis and metabolism of structural proteins, lipids, glycans, and signaling molecules, as well as an integral component of the immune, nervous, and endocrine systems[1]. As such, injury to the skin and the repair process to the skin is a well-tuned process involving multiple systems and cell types. Wound heading follows an intricately orchestrated process of hemostasis, inflammation, proliferation, epithelialization, and remodeling confined to the injury location[2]. In addition, fibroblasts and fibroblast-secreted materials are involved in every step of the process.

Wound treatment forms a significant burden on the healthcare system and destroys the quality of life for the victims of non-healing wounds. Difficult to heal wounds, chronic wounds are persistent, time-consuming, and costly to treat. In addition, injury to the skin due to diseases such as diabetes or cancer, cuts, abrasions, bedsores, or burns can have a lasting impact on a patient's physical, emotional, and psychological well-being. While some injuries caused to the skin can heal normally and quickly, certain underlying health aspects, such as age, health status, and certain diseases, can adversely impact the intricately orchestrated wound healing process, thereby requiring external intervention to heal properly. Since fibroblasts are an integral part of every step in the wound healing process and externally applied single-cell or spheroid fibroblasts and/or fibroblast-derived products on wounds can initiate, maintain, and accelerate the wound healing process in diabetic ulcers, bedsores, major and minor cuts, surgical excisions, or incisions (including large), amputation, biopsies, and burns. Their functionality in the repair of the wound should be considered integral. For large wounds or burns where the closure of the wound is impaired or reduced, fibroblasts, exosomes, conditioned media, lysates, or apoptotic bodies and/or fibroblast-related products can be used to produce the key extracellular matrix (ECM) proteins such as laminins, fibronectins, collagens, and elastic fibers, as well as to produce many of the growth factors and cytokines necessary for efficient wound healing such as bFGF, VEGF, HGF, PDGF, TGF-B1, KGF, IL-6, IL-8 and inhibitors of metalloproteinases to create a new skin during the healing process to hasten healing and/or prevent infection[3-5].

### BRIEF SUMMARY

Embodiments of the disclosure encompass compositions, methods, and systems for introducing activated or inactivated single-cell or spheroid fibroblasts, exosomes, lysates, conditioned media, or apoptotic bodies and/or fibroblast-related products onto a synthetic organic or inorganic polymer or biopolymer scaffolding and/or directly onto the boundaries of a wound generating a biologic barrier similar to skin. In particular embodiments, these products will produce particular ECM proteins, such as laminins, fibronectins, collagens, and/or elastic fibers, as well as to produce many of the growth factors and cytokines useful for efficient wound healing, such as bFGF, VEGF, HGF, PDGF, TGF-B1, KGF, IL-6, IL-8, and/or one or more inhibitors of metalloproteinases. This will result in recruitment of cells needed for the healing process while protecting the wound site and/or preventing infection. In particular embodiments, the disclosure concerns the process of seeding fibroblast cells onto and/or into a synthetic organic or inorganic polymer or biopolymer scaffolding, with or without perforations to generate a skin-like covering to be used on any type of wound, including at least large non-healing wounds or for burn victims. The wound may be non-intentional, such as generated by ill health or an accident, or the wound may be intentional, such as an excision or incision made as part of a medical procedure.

Single-cell or spheroid fibroblasts, exosomes, lysates, conditioned media from fibroblasts, and/or apoptotic bodies and/or fibroblast-related products for use in any methods of the disclosure may be exposed to certain medium component(s) in specific embodiments, in certain embodiments.

In specific embodiments, there is an *in vitro* method of producing activated or unactivated single-cell or spheroid fibroblasts, exosomes from fibroblasts, lysate from fibroblasts, apoptotic bodies from fibroblasts, and/or fibroblast-related products for introduction into and/or onto a synthetic polymer and/or biopolymer scaffolding or biologic mesh (as examples of substrates) to generate a material to cover at least one wound and/or at least one burn of an individual.

The foregoing has outlined rather broadly the features and technical advantages of the present invention so that the detailed description of the invention that follows may be better understood. Additional features and advantages of the invention will be described hereinafter, which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages, will be better understood from the following description when considered in connection with the accompanying figures. It is to be expressly understood, however, that each of the figures is provided for the purpose of illustration and description only and is not intended as a definition of the limits of the present disclosure.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.
FIG. 1A demonstrates the diet components of the mice model of human metabolic syndrome and obesity-induced type 2 diabetes.
FIG. 1B shows attainment of desired baseline blood glucose levels over time.
FIG. 2 provides one example of a regiment of wound treatment.
FIG. 3 demonstrates day-to-day wound closure progression when comparing control Matrigel with topically provided fibroblasts (FB) (top image where the control bar is left of the FB bar); control Matrigel compared to FB-derived exosomes (bottom left image where the control bar is left of the FB bar); and control Matrigel compared to subcutaneously provided FB (bottom right image where the control bar is to the left of the FB bar).
FIG. 4 shows progression of wound closure comparing control Matrigel (top line) with subcutaneously provided fibroblasts (second line from the top), fibroblast exosomes (third line from the top), and topically provided fibroblasts (bottom line).
FIG. 5 shows representative images of wound healing and closure.
FIG. 6 shows one example of an experiment design to examine the effects of freeze/thaw storage on fibroblast exosomes and fibroblast lysate.
FIG. 7 demonstrates the impact of frozen/thawed fibroblast exosomes and fibroblast lysate.
FIG. 8 demonstrates day-to-day wound closure progression :freeze/thaw embodiments.
FIG. 9 shows wound closure with fibroblast and fibroblast-derived exosomes.
FIG. 10 shows an example of a test of wound cover products on cell viability, particularly a test of 3M Nexcare^{®} and ElaSkin^{®} on mouse dermal fibroblast cells.
FIG. 11 shows the impact of growth and cell size as compared to control untreated cells.
FIG. 12 provides one example of a study design to examine the effects of fibroblast spheroids on wound healing covered with 3M Nexcare^{®}.
FIG. 13 demonstrates fibroblast spheroids tested with 3M Nexcare^{®} wound cover. In the left image, the Spheroids + 3M Nexcare^{®} line is the bottom line. On the right image, the Spheroids + 3M Nexcare^{®} bar is left of the 3M Nexcare^{®} control bar.
FIG. 14 provides representative images of wound healing with spheroids covered with 3M Nexcare^{™}.

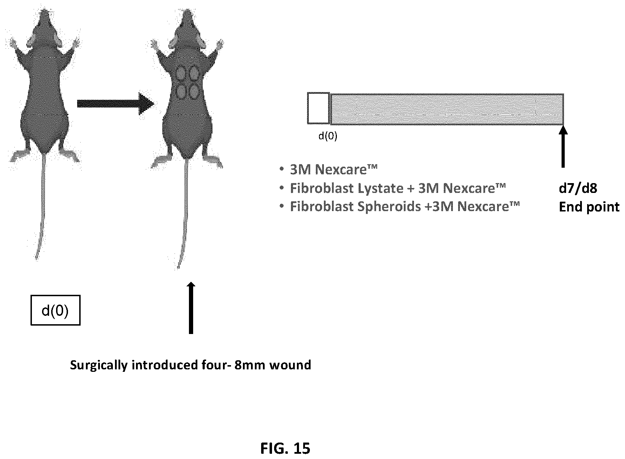

FIG. 15 provides one example of a study design to examine the effects of fibroblast spheroids and lysate on wound healing in wild type mice.

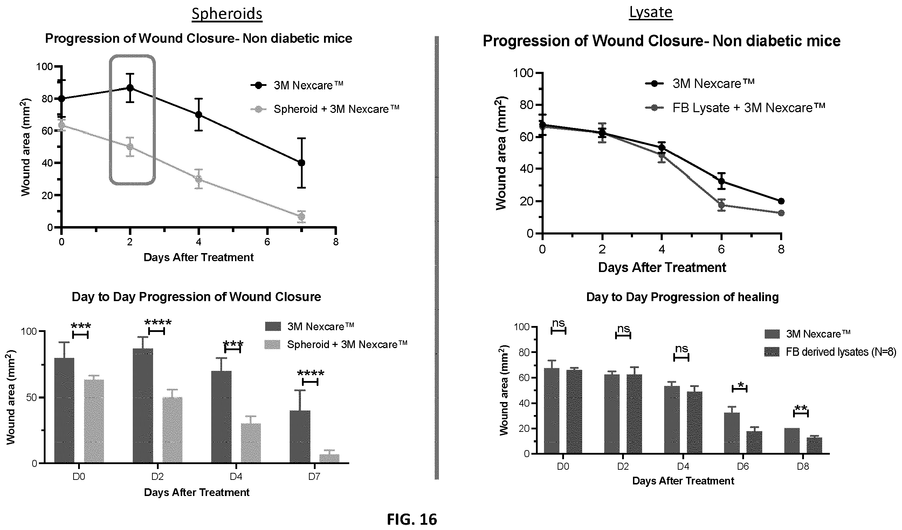

FIG. 16 demonstrates the effect of spheroids and lysate with 3M Nexcare^{®} on wild type mice. In the top left image, Spheroids + 3M Nexcare^{®} is the lower line. In the bottom left image, the Spheroids + 3M Nexcare^{®} bar is to the right of the 3M Nexcare^{®} bar. In the upper right image, the FB Lysate + 3M Nexcare^{®} line is below the 3M Nexcare^{®} line. In the bottom right image, the FB derived lysates + 3M Nexcare^{®} bar is to the right of the control 3M Nexcare^{®} bar.
FIG. 17 provides images for fibroblast spheroids with 3M Nexcare^{®} cover vs. 3M Nexcare^{®} cover only in wild type mice.
FIG. 18 provides representative images showing the effect of fibroblast lysate on wild-type mouse wound closure with 3M Nexcare^{®} cover.
FIG. 19 provides one example of a study design to examine the effects of one-time treatment with mouse fibroblast spheroids, human fibroblast spheroids +3M Nexcare^{®}, compared with Grafix^{®}.
FIG. 20 provides mouse and human fibroblast spheroids wound healing progression comparison with Grafix^{®} in diabetic mice.
FIG. 21 provides representative images of wound closure with fibroblast spheroids compared to Grafix^{®}.
FIG. 22 shows an example of fibroblast spheroids implantation, migration, and proliferation on wound surface.
Fig. 23 are photographs showing treatment by a control (top line), topical administered human dermal fibroblasts spheroids (middle line), or a competitor treatment (bottom line) over 24 days.
Fig. 24 is a graph that shows percentage change in chronic wound area post treatment with a control or an HDF derived spheroids with Nexcare^{™}. The top data line is the control and the bottom data line with the HDF derived spheroids plus Nexcare^{™} treatment.
Fig. 25 is a graph of INF-γ levels at days 7 and 17 in skin tissue and serum that have been treated with HDF spheroids or a control.
Fig. 26 is a graph of EGFR levels at days 7 and 17 in skin tissue and serum that have been treated with HDF spheroids or a control.
Fig. 27 is a graph of VEGF levels at days 7 and 17 in skin tissue that has been treated with HDF spheroids or a control.
Fig. 28 is a graph of IL-10 levels at days 7 and 17 in skin tissue and serum that have been treated with HDF spheroids or a control.
Fig. 29 is a graph of IL-1a levels at days 7 and 17 in skin tissue and serum that have been treated with HDF spheroids or a control.
Fig. 30 is a graph of IL-6 levels at days 7 and 17 in skin tissue and serum that have been treated with HDF spheroids or a control.

### DETAILED DESCRIPTION

### I. Examples of Definitions

In keeping with long-standing patent law conventions, the words "a" and "an" when used in the present specification in concert with the word comprising, including the claims, denote "one or more." Some embodiments of the disclosure may consist of or consist essentially of one or more elements, method steps, and/or methods of the disclosure. It is contemplated that any method or composition described herein can be implemented with respect to any other method or composition described herein.

As used herein, the term "about" or "approximately" refers to a quantity, level, value, number, frequency, percentage, dimension, size, amount, weight, or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular embodiments, the terms "about" or "approximately" when preceding a numerical value indicates the value plus or minus a range of 15%, 10%, 5%, or 1%. With respect to biological systems or processes, the term can mean within an order of magnitude, preferably within 5-fold, and more preferably within 2-fold, of a value. Unless otherwise stated, the term "about" means within an acceptable error range for the particular value.

As used herein, the term "activated" refers to cells treated in vivo, or ex vivo with one or more stimuli capable of inducing one or more alterations in the cell: metabolic, immunological, epigenetic, CRISPR, growth factor secreting, surface marker expression, and production and excretion of microvesicles.

As used herein, the term "activated immune cells" refers to immune cells treated with one or more stimuli capable of inducing one or more alterations in the cell: metabolic, immunological, epigenetic, growth factor secreting, surface marker expression, and production and excretion of microvesicles.

The term "administered" or "administering," as used herein, refers to any method of providing a composition to an individual such that the composition has its intended effect on the patient. For example, one method of administering is by an indirect mechanism using a medical device such as, but not limited to, a catheter, applicator gun, syringe, gel matrix, and a 3D matrix containing one or more cell types and or growth factors and or antibiotics, *etc.* A second exemplary method of administering is by a direct mechanism such as local tissue administration, oral ingestion, transdermal patch, topical, inhalation, suppository, *etc.*

As used herein, "allogeneic" refers to tissues or cells from another body that, in a natural setting, are immunologically incompatible or capable of being immunologically incompatible, although from one or more individuals of the same species.

As used herein, "autologous" refers to tissues or cells that are derived or transferred from the same individual's body (*i.e.,* autologous blood donation; an autologous bone marrow transplant).

As used herein, "agent" refers to nucleic acids, cytokines, chemokines, transcription factors, epigenetics factors, growth factors, or hormones.

As used herein, "xenogeneic" refers to tissues or cells from a species different from the patient.

"Cell culture" is an artificial *in vitro* system containing viable cells, whether quiescent, senescent or (actively) dividing. In a cell culture, cells are grown and maintained at an appropriate temperature, typically a temperature of 37°C and under an atmosphere typically containing oxygen and CO₂. Culture conditions may vary widely for each cell type, though, and variation of conditions for a particular cell type can result in different phenotypes being expressed. The most commonly varied factor in culture systems is the growth medium. Growth media can vary in concentration of nutrients, growth factors, and the presence of other components. The growth factors used to supplement media are often derived from animal blood, such as calf serum.

Throughout this specification, unless the context requires otherwise, the words "comprise," "comprises" and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory and that no other elements may be present. By "consisting essentially of" is meant including any elements listed after the phrase and limited to other elements that do not interfere with or contribute to the activity or action specified in the disclosure for the listed elements. Thus, the phrase "consisting essentially of" indicates that the listed elements are required or mandatory but that no other elements are optional and may or may not be present depending upon whether or not they affect the activity or action of the listed elements.

The term "individual," as used herein, refers to a human or animal that may or may not be housed in a medical facility and may be treated as an outpatient of a medical facility. The individual may be receiving one or more medical compositions *via* the internet. An individual may comprise any age of a human or non-human animal and therefore includes both adults and juveniles (*i.e.,* children) and infants. It is not intended that the term "individual" connotes a need for medical treatment, therefore, an individual may voluntarily or involuntarily be part of experimentation, whether clinical or in support of basic science studies. The term "subject" or "individual" may be used interchangeably and refers to any organism or animal subject that is an object of a method or material, including mammals, *e.g.,* humans, laboratory animals (*e.g.,* primates, rats, mice, rabbits), livestock (*e.g.,* cows, sheep, goats, pigs, turkeys, and chickens), household pets (*e.g.,* dogs, cats, and rodents), horses, and transgenic non-human animals.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present disclosure. Thus, the appearances of the foregoing phrases in various places throughout this specification are not necessarily all referring to the same embodiment. Furthermore, the particular features, structures, or characteristics may be combined in any suitable manner in one or more embodiments.

The terms "reduce," "inhibit," "diminish," "suppress," "decrease," "prevent," and grammatical equivalents (including "lower," "smaller," *etc.*) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one embodiment, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

As used herein, the term "resorbable" may refer to the capacity of part or all of a composition to undergo biodegradation (chemical breakdown by biological agent) and in at least some cases the degradation products removed by cellular activity in a biological environment.

As used herein, the term "scaffold" may denote a biocompatible and (in at least some cases) bioresorbable structure that is capable of being implanted in the body or provided on the body in order to provide support and/or promote cell adhesion and/or tissue regeneration.

As used herein, the term "transplantation" refers to the process of taking living tissue or cells and implanting it in another part of the body or into another body.

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression, including a reduction in the severity of at least one symptom of the disease. For example, a disclosed method for reducing the immunogenicity of cells is considered to be a treatment if there is a detectable reduction in the immunogenicity of cells when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition but an improvement in the outlook of a disease or condition. In specific embodiments, treatment refers to the lessening in severity or extent of at least one symptom and may alternatively or in addition refer to a delay in the onset of at least one symptom.

In specific embodiments, externally-applied fibroblasts and/or fibroblast-derived materials on wounds can initiate, maintain, and accelerate the wound-healing process in wounds of any kind, including diabetic ulcers, non-healing wounds, surgical incisions, traumatic injuries, abrasions, skin disorders, cuts, and/or burns. As used herein, the term "wound" encompasses an injury to living tissue caused by a cut, blow, or other impact, typically one in which the skin is cut or broken. In specific embodiments, all of the above-mentioned conditions are wounds that would require healing.

The term "fibroblast-derived material" as used herein refers to fibroblast cell fragments, exosomes secreted from fibroblasts, and/or fibroblast lysate.

The term "biopolymer" can be comprised of one or more or a combination of the following biopolymers such as keratin, fibrin, collagen, cellulose, alginate, chitosan, cyanoflan, hyaluronic acid, and hydrogels, as examples.

### II. Compositions of the Disclosure

The present disclosure concerns compositions for wound, abrasion, cut, puncture, rash or burn healing of any kind. In particular embodiments, the compositions comprise a biologic component and a substrate component that, in specific embodiments acts to deliver the biologic component. The biologic component and substrate component may be delivered to the wound, abrasion, cut, puncture, rash or burn site(s) of the individual already as one object, or they may be delivered separately to the site(s) in need. In specific embodiments when the biologic component and substrate component are delivered separately to the site, the substrate component may be placed at and/or adjacent to the wound site prior to delivery of the biologic component. In some embodiments, the biologic component and substrate component are delivered to the site at substantially the same time.

### A. Biologic Component

Embodiments of the disclosure encompass use of cells and/or cell products as biologic components for wound or burn healing, including to initiate and/or maintain wound healing. In some embodiments, the cells and/or cell products prior to, or during, use for wound healing are exposed to one or more compositions and/or environments to enhance their use in wound healing. In specific embodiments, one or more compositions of the disclosure include one or more biological components of (a) single-cell fibroblasts, spheroid fibroblasts, exosomes from fibroblasts, lysate from fibroblasts, apoptotic bodies from fibroblasts, conditioned media from fibroblasts, and/or fibroblast-related products or any combination thereof. In some embodiments, one or more compositions are utilized at the same wound or burn and may or may not employ the same type of biologic component.

In specific embodiments, cells and/or cell products are exposed in an *in vitro* setting to produce activated fibroblasts, fibroblast-derived materials from activated fibroblasts, activated immune cells, activated keratinocytes, activated basal epithelial cells, activated myofibroblasts or fibroblast derivatives therefrom, including for introduction into, on top of, and/or adjacent to one or more wounds to initiate and or maintain wound healing. In some embodiments, cells that are used have not been activated.

Embodiments of the disclosure include the introduction of fibroblasts or fibroblast-derived materials, fibroblasts activated with one or more agents, and/or fibroblasts (including myofibroblasts or dermal fibroblasts) plus one or more types of immune cells, keratinocytes, and/or basal epithelial cells and/or derivatives thereof into, on top of, and/or adjacent to the wound for the purpose of initiating or maintaining wound healing.

In various embodiments, the introduction of fibroblasts and/or fibroblast-derived materials, fibroblasts activated with one or more agents, and/or fibroblasts plus one or more type of immune cells (including derivatives thereof) into, on top of, and/or adjacent to the wound site results in (1) tissue differentiation; (2) cell recruitment and differentiation of stems cells at the wound site; (3) expansion of already locally present keratocytes, basal epithelial cells, myofibroblasts, and/or dermal fibroblasts; and/or (4) attraction of other cells or biologic processes to aid in the healing, all for the purpose of initiating or maintaining wound healing.

In particular embodiments, fibroblasts or other cells encompassed herein become activated following exposure to one or more conditions and/or agents. Specific agents that may modify the fibroblasts include one or more of nucleic acids, cytokines, chemokines, growth factors, and/or exosomes prior to and/or during their use. The fibroblast cells may be activated, such as having activated or engineered surface markers, nucleic acid modification, and/or expression or excretion of one or more chemokines, one or more cytokines, exosomes, and/or one or more growth factors. The cells may be activated by one or more chemical agents, RNA, micro RNA, RNAi , DNA, viral nucleic acid, and/or exosomes. In specific embodiments, the cytokine is selected from the group consisting of IFN-gamma, TNF-alpha, interleukin(IL)-l, IL-6, IL-7, IL-8, IL-12, IL-15, IL-17, IL-33, and a combination thereof. In specific embodiments, the growth factor is selected from the group consisting of FGF-l, VEGF, and a combination thereof. In specific embodiments, the cells are activated following exposure to human platelet-rich plasma, platelet lysate, umbilical cord blood serum, autologous serum, human serum, serum replacement, or a combination thereof. In specific embodiments, the cells are activated following exposure to hypoxia. The hypoxia may be 0.1%-10%, 0.1%-5%, 0.1%-2.5%, or 0.1%-1% oxygen, for example. In specific embodiments, the hypoxia occurs for a period of time that is at least or no more than between about 30 minutes-about 3 days, although in some cases, it may be less than 30 minutes or greater than 3 days. The fibroblasts may be exposed to one or more agents prior to and/or during exposure to hypoxia, carbon monoxide, or a combination thereof.

In some embodiments, fibroblasts or fibroblast-derived materials are used with one or more adjuvants to activate the immune system and initiate and or maintain the wound-healing process. These adjuvants could be chemical, mechanical, viral, genetically modified components or bacterial products-based.

In specific embodiments, encapsulated RNA, microRNA, or RNAi, and/or fibroblast-derived exosomes or other microvesicles are utilized by any other cell or cell fragments derived from fibroblasts, with fibroblasts locally initiating and maintaining the wound-healing process.

Embodiments of the disclosure include the use of fibroblasts or fibroblast-derived materials in angiogenesis formation, repair, and regeneration in a wound site and surrounding tissue.

Embodiments of the disclosure include *in vitro* methods of producing activated or unactivated single-cell or spheroid fibroblasts, exosomes from fibroblasts, lysate from fibroblasts, apoptotic bodies from fibroblasts, and/or fibroblast-related products for introduction into and/or onto a synthetic polymer or biopolymer scaffolding or biologic mesh to generate a material to cover at least one wound and/or at least one burn of an individual. In specific embodiments, the material is a fabric, such as a skin-like fabric. In particular embodiments, the method is directly onto the patient. The single-cell or spheroid fibroblast, exosomes from fibroblasts, lysate from fibroblasts, apoptotic bodies from fibroblasts, and/or fibroblast-related products may be autologous, allogeneic, syngeneic, or xenogeneic with respect to the individual.

Embodiments of the disclosure include use of inorganic polymer such as PDMS, HPE, and direct culturing of fibroblast spheroids in perforations of the inorganic polymer.

Embodiments of the disclosure include use of inorganic polymer such as PDMS, HPE and dispensing of fibroblast cells into perforations for seeding and culturing into spheroids.

Embodiments of the disclosure include use of inorganic polymer such as PDMS, HPE as a means of placing activated or unactivated single-cell or spheroid fibroblasts, exosomes from fibroblasts, lysate from fibroblasts, apoptotic bodies from fibroblasts, and/or fibroblast-related products onto the surface or into engineered perforations of said polymer for use in wound dressing.

### B. Substrates and Application of Biologic Component(s)

In some embodiments, one or more biologic components are present on, or are delivered on, a substrate comprising the biologic component(s) in the substrate, on the substrate, around the substrate, or a combination thereof. In other embodiments, the biologic component(s) is applied to the substrate at the site of need. In some embodiments, one or more compositions are utilized at the same wound or burn and may or may not employ the same type of substrate.

In specific embodiments of the composition, one or more substrate components are comprised of a synthetic polymer and/or a biopolymer, and in specific cases the biopolymer is a natural polymer. The synthetic polymer and/or a biopolymer may be of any type so long as they are compatible for use on and/or in an individual. In embodiments wherein the substrate of the composition comprises biopolymers such as keratin, fibrin, collagen, cellulose, alginate, chitosan, cyanoflan, hyaluronic acid, and/or hydrogels. In embodiments wherein the polymer comprises one or more synthetic polymers, the synthetic polymer may be poly(ethylene glycol) (PEG)/poly(ethylene oxide)(PEO), poly(vinyl pyrrolidone) (PVP), poly(vinyl alcohol) (PVA), [15], poly(hydroxyethyl methacrylate) (PHEMA), polyurethanes (PUs), poly (α-esters) [e.g., poly(lactic-co-glycolic acid) (PLGA), polyglycolic acid (PGA), polylactide (PLA), and/or poly(ε-caprolactone)(PCL). In embodiments wherein the polymer comprises one or more synthetic inorganic polymers such a PDMS, HPE, natural minerals, synthetic silicates, phosphates, zeolites, clay, mesoporous silica, polyP, and so forth.

The configuration parameters of the substrate, including at least size, substance, thickness, presence of any one or more demarcations, presence of any one or more apertures, shape, *etc.,* may or may not be tailored to an intended wound/burn. In some embodiments, the substrate is tailored in configuration parameter(s) to the intended use of the composition, whereas in other cases the substrate is utilized in an off-the-shelf manner. In cases wherein the substrate is tailored for a specific use, the biologic component may or may not already be present on the substrate. In cases wherein the substrate is not initially tailored and is to be utilized in an off-the-shelf manner, the biologic component may or may not already be present on the substrate.

The shape of the outside border of the substrate may be random or may be of a particular shape, such as a square, rectangle, triangle, circle, trapezoid, rhombus, crescent, and so forth. Thus, the substrate may be a particular geometric formation. The width of the substrate that is applied at the site may depend on the width of the wound. The substrate may be reduced in size, such as tailored to the wound, prior to or after placement of the substrate at or on the wound. The size and shape of the substrate can be adjusted to wound size.

The substate may be a scaffolding, in some embodiments. In specific embodiments, the scaffolding as a structure that facilitates placement of the cells in single cell or spheroid format, inside the scaffolding, and allow for cell migration and/or cell proliferation, for example at the dermal layer, from the location where the cells are located. The scaffolding allows tissue growth, such as to mimic the biological process or structure for tissue engineering that is in need of being replaced, in some embodiments. The scaffolding may be fibrous, porous, or of a hydrogel, in specific embodiments.

In specific cases, the substrate is a tray, a cup, a mesh, and so forth. The substrate may be comprised of a biopolymer, organic or inorganic polymer fabric of any kind.

In specific embodiments, the substrate comprises one or multiple apertures, or perforations. The apertures, or perforations with respect to the substrate may be in a random pattern or in an ordered pattern. The size of the apertures may be configured to be big enough to allow efficient cell migration and to allow securing of one or more spheroids at the site.

In specific embodiments, there is introduction of single-cell or spheroid fibroblasts, exosomes from fibroblasts, lysate from fibroblasts, apoptotic bodies from fibroblasts, and/or fibroblast-related products or any combination thereof onto a substrate of any kind, such as a synthetic polymer or biopolymer scaffolding, tray, geometric formation, biologic fabric, mesh, or polymer fabric. The step(s) of applying the biologic component(s) to the substrate component, whether or not it is prior to placement at the wound or burn or at the time of placement at the wound or burn, may be of any suitable manner.

In specific embodiments, there is introduction of single-cell or spheroid fibroblasts, exosomes from fibroblasts, lysate from fibroblasts, or apoptotic bodies from fibroblasts and/or fibroblast-related products onto a synthetic polymer or biopolymer scaffolding, tray, geometric formation, biologic fabric, mesh, or polymer fabric using a device, such as a spreading device. Any spreading device referred to herein may be considered as an applicator.

In certain embodiments, there is introduction of single-cell or spheroid fibroblasts, exosomes from fibroblasts , lysate from fibroblasts, or apoptotic bodies from fibroblasts and/or fibroblast-related products onto a synthetic polymer or biopolymer scaffolding, tray, geometric formation, biologic fabric, mesh, or polymer fabric using a device, such as a spreading device, designed with one or more grooves.

In certain embodiments, there is introduction of single-cell or spheroid fibroblasts, exosomes from fibroblasts, lysate from fibroblasts or apoptotic bodies from fibroblasts and/or fibroblast-related products onto a synthetic polymer or biopolymer scaffolding, tray, geometric formation, biologic fabric, mesh, or polymer fabric using a device, such as a spreading device. In certain embodiments, the device is designed with an ordered pattern of holes to allow spheroid placement or with a random series of holes to allow randomized spheroid placement.

In some embodiments, there is introduction of single-cell or spheroid fibroblasts, exosomes from fibroblasts, lysate from fibroblasts, or apoptotic bodies from fibroblasts and/or fibroblast-related products onto a synthetic polymer or biopolymer scaffolding, tray, geometric formation, biologic fabric, mesh, or polymer fabric using a spray or misting device. In specific embodiments, the spray or misting device is utilized for uniformity of placement of the biological material onto a substrate.

In certain embodiments, there is introduction of single-cell or spheroid fibroblasts, exosomes from fibroblasts, lysate from fibroblasts, or apoptotic bodies from fibroblasts and/or fibroblast-related products onto a substrate comprising a synthetic polymer and/or biopolymer, such as a scaffolding, tray, geometric formation, biologic fabric, mesh, or polymer fabric, using a 3-D printer.

In specific embodiments, there is culturing of fibroblast spheroids inside the holes of a perforated synthetic or biopolymer in which the apertures are spaced in such a way as to accommodate the proliferation from a specific size and fibroblast cell number-containing spheroid onto a wound surface.

In some embodiments, there is dispensing of specific-sized fibroblast spheroids into the apertures of a synthetic and/or biopolymer substrate such that only one spheroid is placed in a single aperture to allow the proliferation of the fibroblasts onto the wound surface from the opposite side of the aperture.

In particular embodiments, there is use of a high-resolution camera to generate an image for the introduction of single-cell or spheroid fibroblasts, exosomes from fibroblasts, lysate from fibroblasts, or apoptotic bodies from fibroblasts and/or fibroblast-related products in a 3-D printer for the purpose of matching the boundaries of the wound being covered.

### EXAMPLES

The following examples are included to demonstrate particular embodiments of the disclosure. It should be appreciated by those of skill in the art that the techniques disclosed in the examples that follow represent techniques that function well in the practice of the invention and thus can be considered to constitute particular modes for its practice. However, those of skill in the art should, in light of the present disclosure, appreciate that many changes can be made in the specific embodiments that are disclosed and still obtain a like or similar result without departing from the spirit and scope of the subject matter of the disclosure.

### EXAMPLE 1

### THERAPEUTIC USE OF FIBROBLASTS FOR USE IN WOUND HEALING

### Introduction:

Skin wound repair is essential for organismal survival and failure of skin would repair, which leads to non-healing wounds, is a leading health issue worldwide. Wound healing studies are intricate, mainly because of the wound environment's multifaceted nature and the healing process's complexity, which integrates a variety of cells and repair phases, including inflammation, proliferation, re-epithelialization, and remodeling. Delayed foot wound healing is a significant complication attributed to hyperglycemia in type 2 diabetes mellitus (DM) patients, and these wounds may develop into foot ulcers. Studies have shown that diabetic wounds heal less than 50% compared to non-diabetic wounds 30 days after they have encountered the wounds.

Multiple factors are responsible for the delay in wound healing. These include impaired fibroblast proliferation and migration, hyperglycemia, and impaired keratinocyte proliferation and migration. Another major cause of impaired wound healing is a sub-optimal immune response, increased levels of reactive oxygen species, deregulated angiogenesis, and persistent bacterial colonization at the site of the wound. Dermal fibroblasts are associated with every stage of the wound healing including hemostasis, inflammation, proliferation, and remodeling. In addition, the mechanistic understanding of chronic wounds has been a significant challenge due to the lack of appropriate genetic mouse models. The present disclosure demonstrates the effect of fibroblasts in diabetes-induced ulcers and illustrates accelerated wound healing with fibroblasts and fibroblast-derived materials.

### Methods and materials

### Mice:

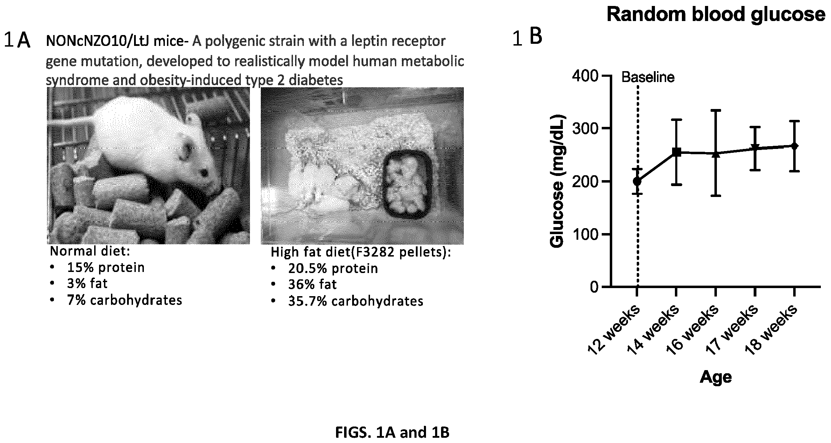

Polygenic model (Leptin gene, Leptin receptor (LEPR), A^{y} gene mutation)-NONcNZO10/LtJ mice at 10-12 weeks from Jacksons Laboratory were used for the study. Mice were housed in individually vented cages on a 12-h light/dark cycle. Once the desired baseline blood glucose levels of 300 mg/dL were reached (FIG. 1B), mice were placed on high-fat feeding (F3282 pellets) containing 36% fat (FIG. 1A).

### Cell culture:

Murine embryo Fibroblasts were obtained from American Type Culture Collection (ATCC^{®}) and were cultured in DMEM High Glucose and supplemented with 10% Fetal bovine serum (FBS) and 1% penicillin-streptomycin, respectively. Cells were incubated at 37°C in 5% CO₂.

### Fibroblast-derived Exosomes Isolation:

### Conditioned medium preparation

Fibroblasts were plated and cultured in DMEM media with 10% FBS for 24 hours, then washed three times with PBS, and finally cultured in 3ml serum-free DMEM media for 2 hours. The conditioned medium was collected and filtered through a 0.22-µm filter to remove cellular debris.

### Isolation and characterization of exosomes released by fibroblasts

Exosomes were isolated from cultured fibroblasts by using Total Exosome Isolation Kit. In brief, 0.5ml of total exosome isolation reagent was added to each 1ml of filtered conditioned media and mixed well by inverting. After overnight incubation at 4°C, the mixture was centrifuged at 12,000×g for 70min at 4°C, and all supernatant was removed by aspiration. Exosome pellets were resuspended in a very low volume of DMEM medium so as not to dilute prior to use.

### In vivo treatment:

### Cell preparation technique:

Cells will be cultured in T225 flasks at 37°C. Once the cells are 70% confluent, they are trypsinized with 0.5% trypsin and centrifuged at 400g for 5mins. The supernatant was discarded, and the pellets were resuspended in PBS. Cells PBS. 1 × 10⁶ are aliquoted.

### Full thickness wound model:

Mice were anesthetized using inhalation of Isoflurane. Mice were shaved and sterilized with betadine and alcohol. On day 0, 4 full-thickness wounds were created using a sterile 8-mm punch biopsy on the back on either side of the midline at the level of the shoulders. Using serrated forceps, the skin was lifted, and a full-thickness wound through the subcutaneous tissue was created using an iris scissors.

### Wound treatment:

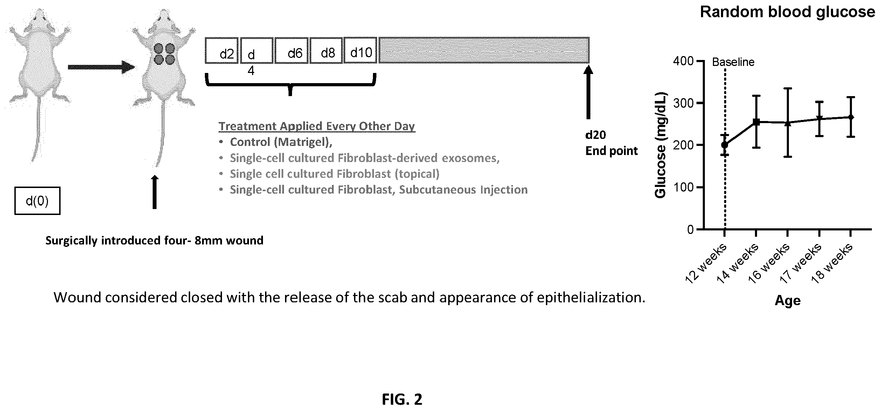

Mice were anesthetized using inhalation of Isoflurane. Mice are grouped into four treatment groups: Matrigel, topical fibroblast (FB (Top)), fibroblast given subcutaneously (FB (SC)), and fibroblast-derived exosomes (FB-derived exosomes). The respective therapeutic compounds are gently smeared on the wound or subcutaneously administered around the wound. All mice received a total of 5 treatments (Day 1, Day 3, Day 5, Day 7, Day 9) (FIG. 2).

### Wound measurement:

The wounds were measured every day. Mice were anesthetized using 5% inhalation of Isoflurane. The wound diameter and area of the wound was measured using an eKare^{®} insight advanced wound imaging device which is also approved for use in the clinic. After measurement, a clean occlusive dressing is re-applied, and the animal is kept warm until fully recovered. The wound closure for the four wounds was averaged per mouse.

### Histology

Skin fragments encompassing the wounded area were excised and utilized for further analysis. For H&E staining, tissue samples are collected on Day 20 and fixed in 10% NBF, and embedded in paraffin. H&E staining was performed with ST Infinity H&E staining system, and Sirius red staining was performed with 0.1% picrosirius red. H&E-stained slides were imaged.

### Immunolabeling of tissues

For immunofluorescence with CD31, tissues were formalin-fixed and paraffin-embedded. Five-micrometer sections were rehydrated, and antigen retrieval was performed in 10 mM citrate buffer (pH 6.0) for 15 minutes to 1 hour. After one hour of blocking with 1% BSA at room temperature, the sections were incubated overnight at 4°C with primary antibody (CD31, Thermo Fisher) and then with secondary antibody (Alexa Fluor 568, goat, anti-rat, Thermo-Fisher) for 1 hour at room temperature.

For paraffin-embedded samples, tissues were fixed in 4% buffered paraformaldehyde (PFA) for 20 min and permeabilized in 0.3% Triton-X for 30 min. After blocking with 1% BSA (30 min at room temperature), the sections were incubated overnight at 4°C with primary antibody ( Cytokeratin 5, Thermo Fisher), followed by secondary antibodies (Alexa Fluor 546, goat anti-rabbit) for 1 hour at room temperature, and 30-min incubation with nuclear stain (DAPI).

### Quantitative real-time PCR

RNA was isolated from snap-frozen tissues and extracted with Trizol reagent according to the manufacturer's instructions. cDNA was synthesized using a High-Capacity cDNA Reverse Transcription Kit. Real-time quantitative PCR was carried out using SYBR Green Real-Time PCR Master Mix.

### Enzyme-Linked Immunosorbent Assay

### Collection of Serum:

Blood was allowed to clot for 2 hours at room temperature before centrifuging for 15mins at 2000g to separate the serum and begin the assay immediately.

Tissue biopsy samples. Biopsy samples of healing wound areas were taken at several time points during the experiment, flash frozen, and stored. Lysates from these tissues were then used to assay for cytokines and growth factors.

### Mouse Cytokine array analysis:

Mouse cytokine antibody arrays were performed using the Abcam Mouse cytokine antibody array (120 targets- ab197461) kit according to the manufacturer's instructions.

### List of cytokines detected in the array analysis:

Tumor necrosis factor (TNF-α),
interleukin-6 (IL-6),
IL-1β,
interferon-gamma (IFN-γ),
IL10;
epidermal growth factor (EGF),
vascular endothelial growth factor (VEGF).

### Results:

### Topical fibroblasts accelerate wound healing in diabetics

To demonstrate that topically administered fibroblasts accelerate wound healing, NONcNZO10/LtJ mice aged 10-12 weeks were administered a high-fat diet with 36% fat after their baseline blood glucose levels reached the desired 300 mg/dL concentration. Once the blood glucose levels reached 300mg/dL, mice were randomized into four treatment groups: Matrigel, topical fibroblast (FB (Top)), fibroblast given subcutaneously (FB (SC)), and fibroblast-derived exosomes (FB-derived exosomes). Four 8mm wounds were created at the back of each of these mice, and they were treated with 50uL of their respective treatments mixed with Matrigel. The diameter of these wounds were measured every other day until the wounds healed, and the mice were euthanized for further analysis. Treatment effectiveness was assessed by plotting the wound diameter and area using the diameter and area data generated by eKare^{®} insight advanced wound imaging device which is also approved for use in the clinic.

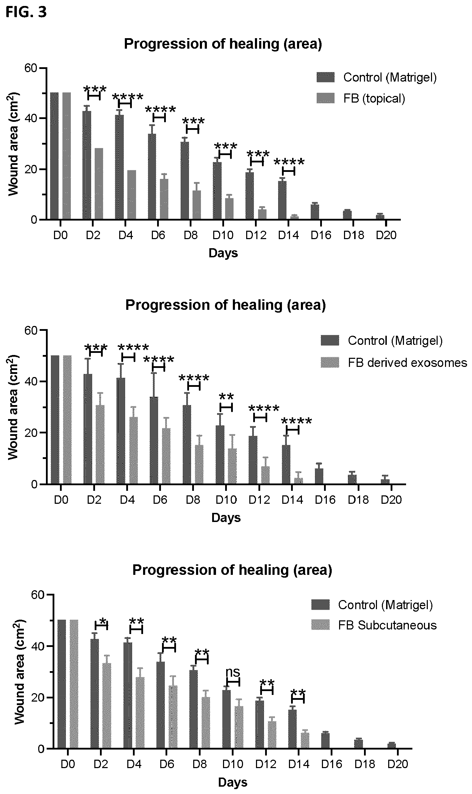

The experiment and the analysis of the captured data confirmed that mice treated with FB (topical) showed an accelerated wound healing rate compared to the control group that received Matrigel only. (FIG. 3) Wound area measurement also revealed a significant improvement in wound size in the mice that received FB (topical), followed by FB-derived exosomes and FB (SC), compared to the control groups. This finding also confirmed that FB (topical) accelerates wound healing in diabetic wounds, followed by fibroblast-derived exosomes.

### Significance of Certain Embodiments

In this study, it is demonstrated that topically administered fibroblasts show a significant increase in the rate of wound healing when compared to the controls. There also seems to be a substantial and significant improvement in mice that received fibroblast-derived exosomes. These results suggest that the direct application of fibroblasts and fibroblast-derived material and their migration and proliferation at the dermal layer accelerates the wound healing rate in diabetic wounds. Passive accumulation of fibroblasts at the wound site, and their well-established role in immune modulation, trigger an immune response that regulates angiogenesis and accelerates the wound healing rate. Fibroblast and fibroblast-derived material increase the healing rate, and the immune modulation of fibroblasts also prevents bacterial colonization, a known factor for delayed healing in diabetic wounds. These findings are a critical validation step in increasing the therapeutic efficacy of a fibroblast treatment modality.

### Conclusion

The continued testing of several treatment modalities for the delivery of fibroblasts and fibroblast-derived materials to the wound site resulted in a significant improvement in healing rates with topically applied fibroblasts and with fibroblast-derived exosomes. The finding addresses the major issue that exists in diabetic wound healing and is useful to cure this chronic disease associated with diabetics.

### EXAMPLE 2

### WOUND CARE

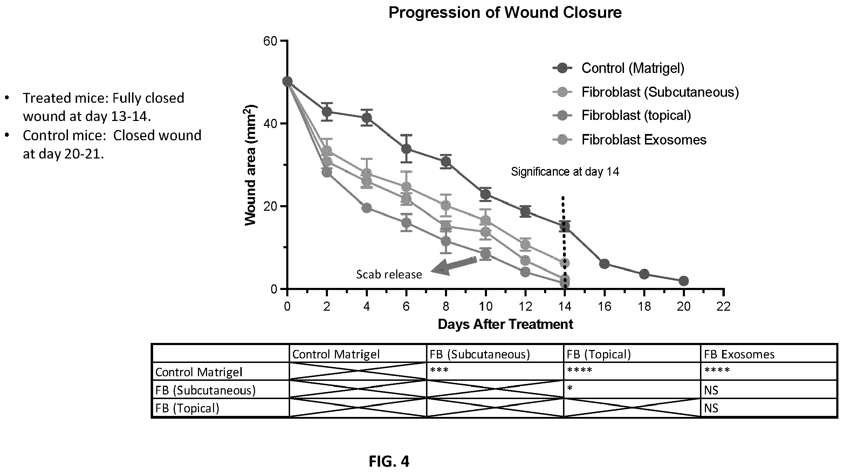

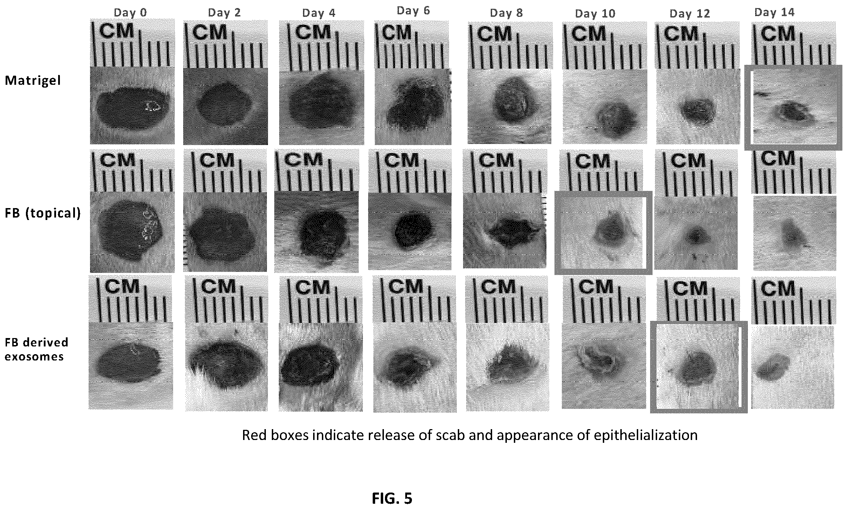

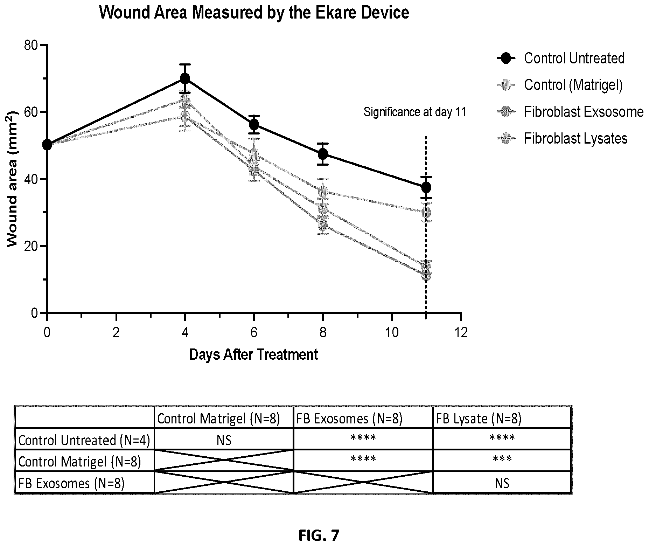

In one in vivo study, the inventors tested single cell fibroblast administration on a wound generated in a diabetic mouse model. One million single cell fibroblasts were either administered topically or subcutaneously around the wound edges. Fibroblast exosomes derived from one million fibroblast media used to grow the single cell fibroblasts were also tested for topical administration. The topical cells and exosomes were mixed with Matrigel^{®} just prior to application so as to keep the cells and the exosomes on the wound. As control, Matrigel^{®} alone was utilized. All control and test samples were administered every other day after measuring the wound diameter and area. The wounds were considered healed once the scab was released from the wound, and epithelialization was visible (FIGS. 2-7).

The results of this study indicated that single cell fibroblasts administered topically, and subcutaneously, as well as fibroblast-derived exosomes, healed the wound significantly faster than the control. In this study, of the three test materials, single cell fibroblasts, and fibroblast-derived exosomes performed significantly better than the subcutaneously-injected fibroblasts.

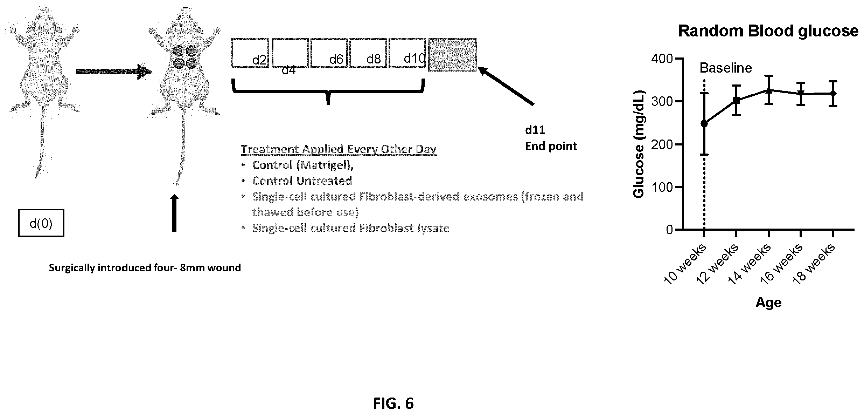

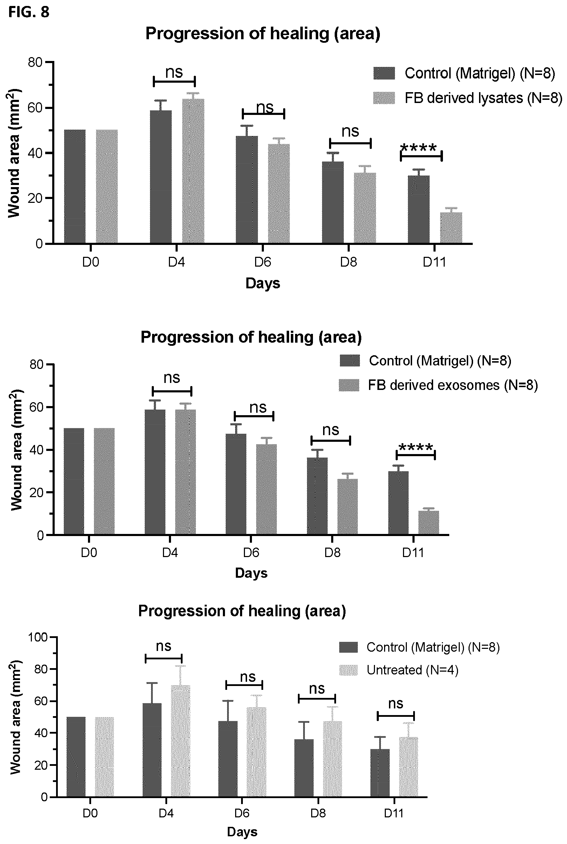

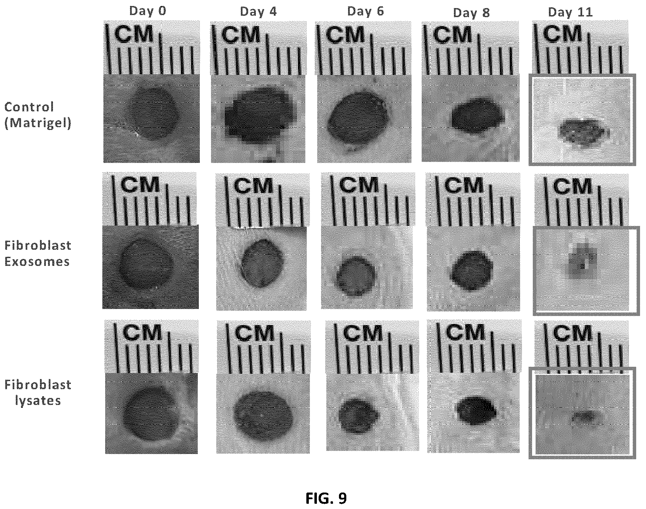

In another study, the purpose of an in vivo experiment was to test the efficacy of single cell fibroblasts, and fibroblast-derived exosomes that have been previously frozen (e.g., in liquid nitrogen) and then thawed just before use. One million single cell fibroblasts were administered topically. Fibroblast exosomes derived from one million fibroblast media used to grow the single cell fibroblasts were also tested for topical administration. The topical cells and exosomes were mixed with Matrigel^{®} just prior to application so as to keep the cells and the exosomes on the wound. As control, Matrigel^{®} alone was utilized. All control and test samples were administered every other day after measuring the wound diameter and area. Wound size was determined every other day using the eKare^{®} device that is clinically approved for use in monitoring chronic wound healing. The eKare^{®} device also takes images of the wounds. The wounds were considered healed once the scab was released from the wound, and epithelialization was visible (see FIGS. 6-9).

The results of this study indicated that frozen/thawed single cell fibroblasts administered topically, as well as the frozen-thawed fibroblast-derived exosomes, healed the wound significantly faster than the control. This study indicated that freeze/thawing of the cells or the exosomes did not impact the efficacy of the materials on wound healing.

In another study, the purpose was to test an alternative, easier method of covering the wounds after fibroblast cell, or fibroblast-derived exosomes, were topically administrated on the wound. Matrigel is an animal product that is not presently approved for human use, so other products that have been approved for use as a wound cover on humans were utilized. For the study, the inventors tested 3M Nexcare^{®} and ElaSkin^{®} by applying the wound cover material in the surface of a culture place, and then inoculating the plate with fibroblast cells and monitoring the growth of the cells in terms of number, as well as cell size, using an instrument called IncuCyte^{®} from Agilent Inc.

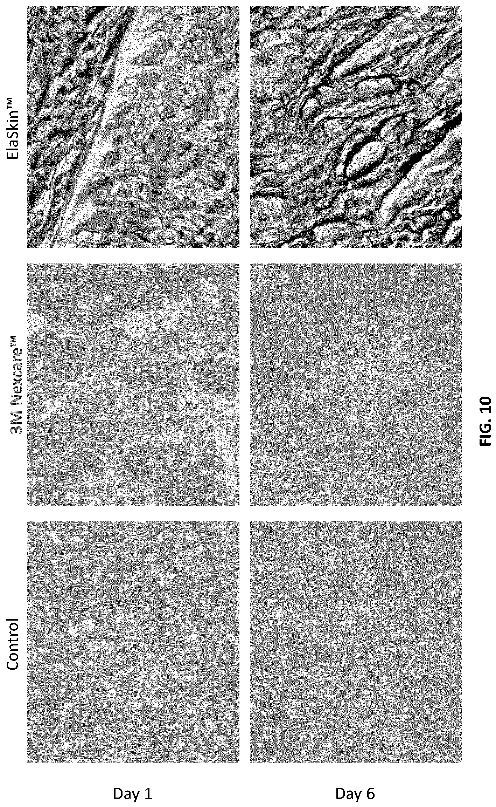

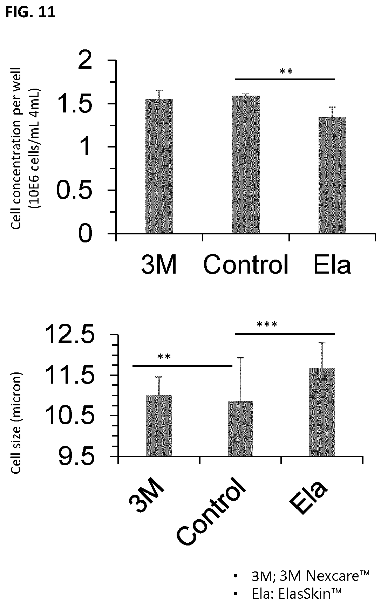

The results of the study indicated that 3M Nexcare^{®} did not impact the growth efficiency or size of the cells, whereas ElaSkin^{®} had a significant impact not only on the growth of the cells, but also on the cell size, indicating adverse impact on cell growth. As a result of this study, 3M Nexcare^{®} was utilized in subsequent studies to cover the fibroblast cells and fibroblast-derived exosomes on the wounds (FIGS. 10-11).

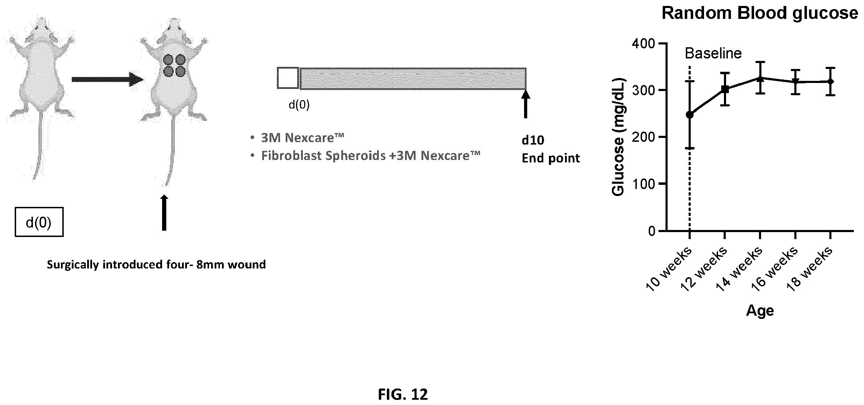

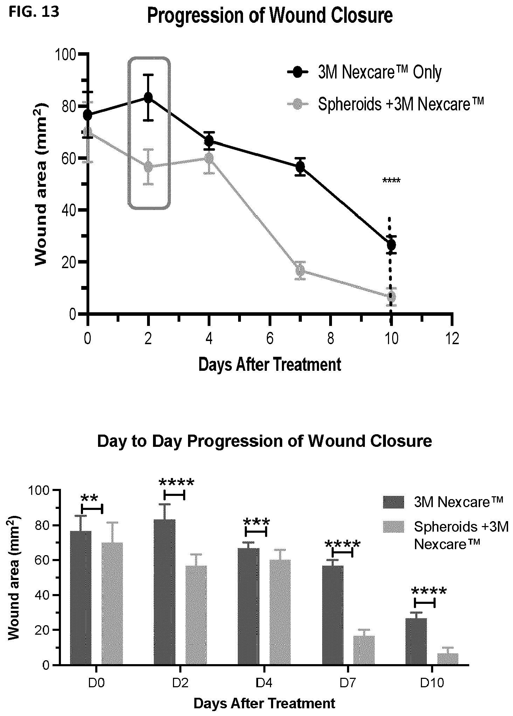

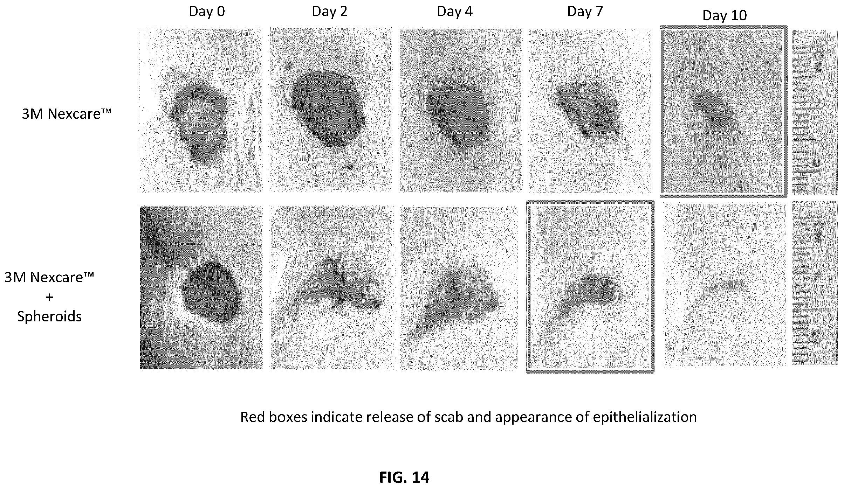

In an additional study, the purpose of the in vivo experiment was to test fibroblast cells grown as a spheroid organoid and its administration on a wound generated in a diabetic mouse model. One hundred fibroblast spheroids, with each spheroid containing approximately 30,000 fibroblast cells (plus or minus of up to 15%), were administered topically on the wound. The wounds were then covered by applying 30-50ml of 3M Nexcare^{®} on the wound . As control only 3M Nexcare^{®} was utilized. The spheroids and control samples were only applied once to be able to test a one-time application of the spheroid fibroblasts. Wound size was determined every other day using the eKare^{®} device that is clinically approved for use in monitoring chronic wound healing. The eKare^{®} device also takes images of the wounds. The wounds were considered healed once the scab was released from the wound, and epithelialization was visible (FIGS. 12-14).

The results of this experiment indicated that spheroid fibroblasts administered healed the wound significantly faster than the control. Not only did the wounds heal approximately three days faster than control, the inflammation of the wound treated with fibroblasts was significantly less than the control.

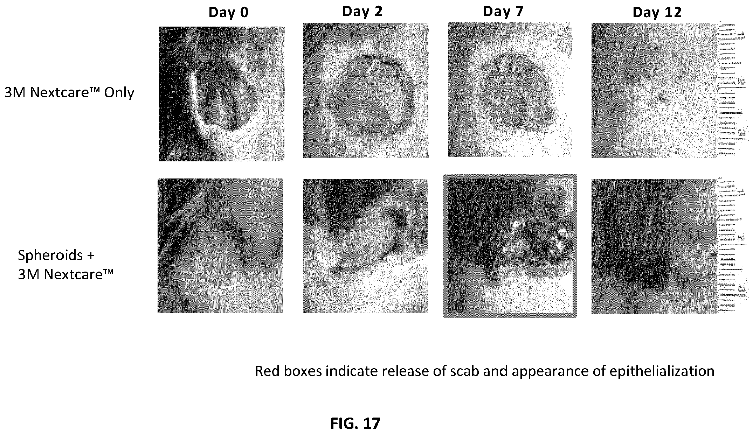

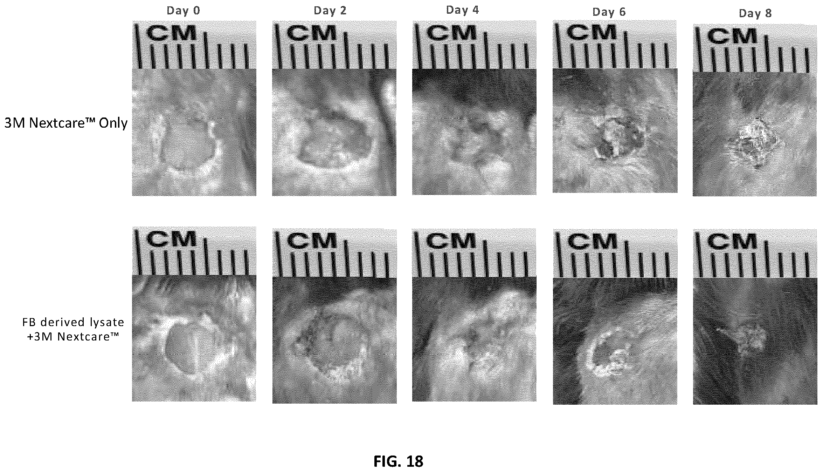

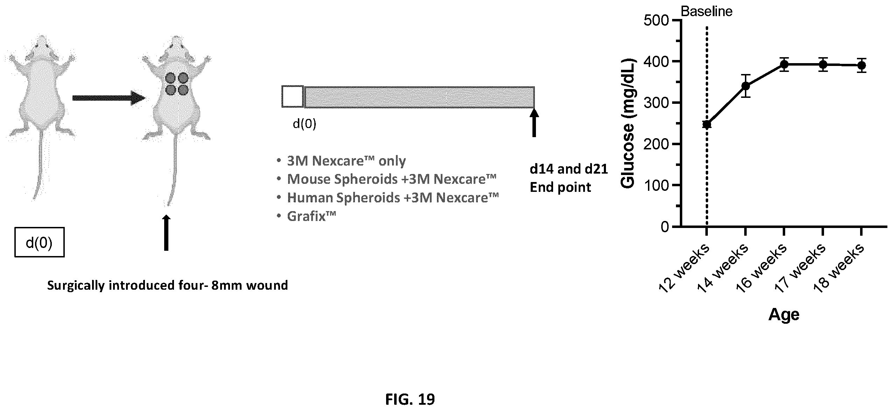

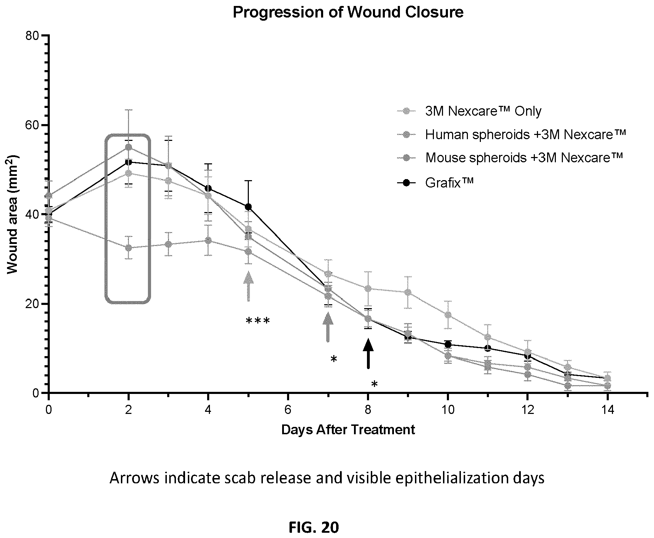

In one study, the purpose of the in vivo experiment was to test fibroblast cells grown as a spheroid organoid, as well as single cell fibroblast-derived lysate and its administration on a wound generated in a wild-type non-diabetic mouse model. One hundred fibroblast spheroids, with each spheroid containing approximately 30,000 fibroblast cells (plus or minus of up to 15%) were administered topically on the wound. Also tested on the wounds was lysate derived from mechanically lysing 1 million single cell fibroblasts. The wounds were then covered by applying 30-50ml of 3M Nexcare^{®} on the wound. As control, the inventors tested only 3M Nexcare^{®}. The spheroids, fibroblast lysate, and control samples were only applied once to test a one-time application of the spheroid fibroblasts, and lysate. Wound size was determined every other day using the eKare^{®} device that is clinically approved for use in monitoring chronic wound healing. The eKare^{®} device also takes images of the wounds. The wounds were considered healed once the scab was released from the wound, and epithelialization was visible (FIGS. 17-20).

The results of this study indicated that spheroid fibroblasts, as well as the fibroblast-derived lysate, healed the wound significantly faster than the control even in non-diabetic wounds. When comparing the fibroblasts spheroids to the lysate, the fibroblast spheroids healed with wound significantly faster than the lysate.

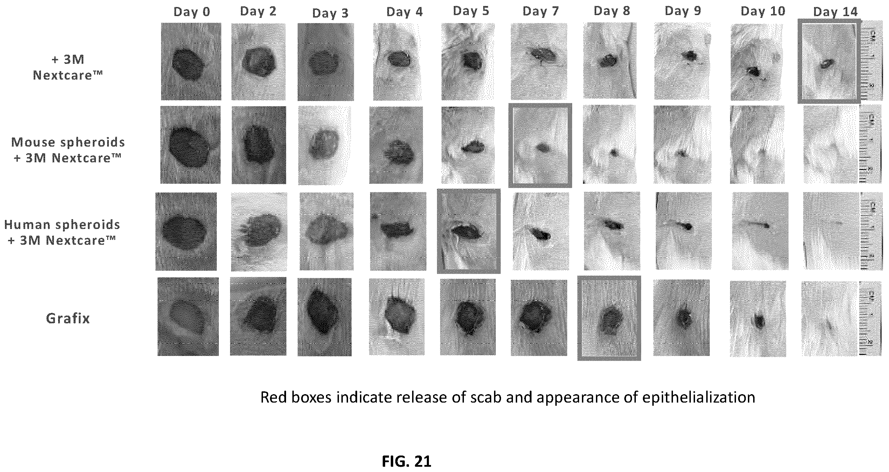

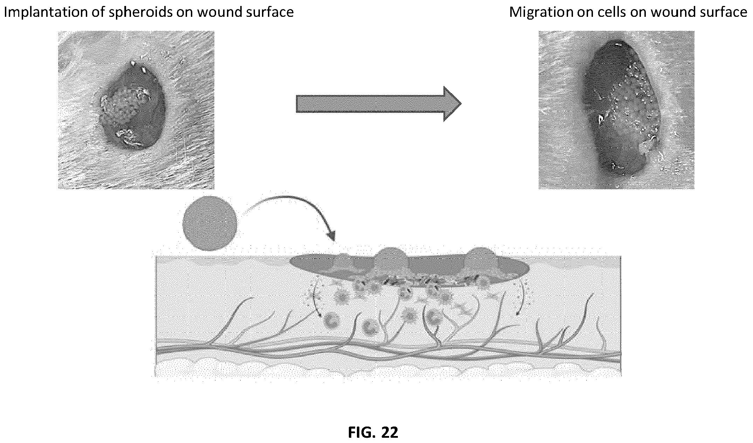

In another study, the purpose of the in vivo study was to test administration on a wound generated in a diabetic mouse model both mouse dermal fibroblasts and human dermal fibroblast cells grown as spheroid organoids. Additionally, the inventors wanted to test a commercially available product called Grafix^{®} which is FDA approved for use in treating chronic wounds. One hundred fibroblast spheroids, with each spheroid containing approximately 30,000 (plus or minus up to 15%) fibroblast cells, were administered topically on the wound. The inventors also tested Grafix^{®} on the wounds by following the direction of application provided in publications for Grafix^{®}. The wounds were then covered by applying 30-50ml of 3M Nexcare^{®} on the wound. As control, the inventors tested only 3M Nexcare^{®}. The human and mouse fibroblast spheroids, Grafix^{®}, and control samples were only applied once to test a one-time application of the spheroid fibroblasts and Grafix^{®}. Wound size was determined every other day using the eKare^{®} device that is clinically approved for use in monitoring chronic wound healing. The eKare^{®} device also takes images of the wounds. The wounds were considered healed once the scab was released from the wound, and epithelialization was visible (FIGS. 21-22).

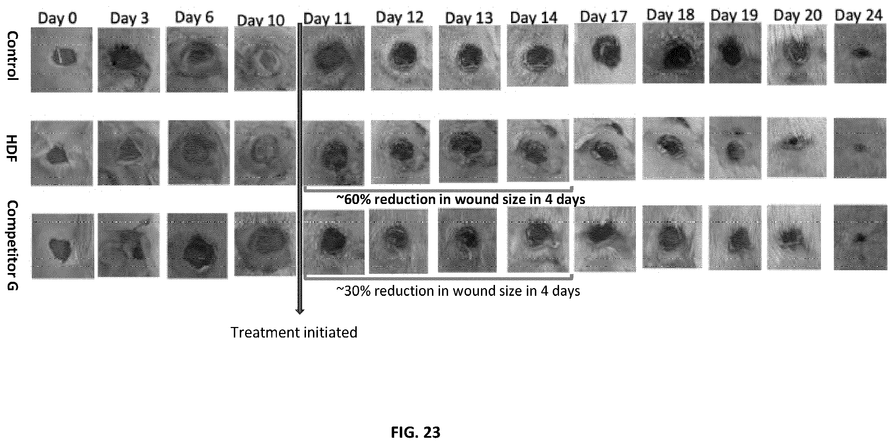

Fig. 23 shows photographs of wounds over 24 days using a control, comparing human dermal fibroblasts spheroids (HDF) to a competitor. From day 11 to day 14 a 60% reduction in wound size is seen in the HDF group, while a 30% reduction in wound size is seen in when using the competitor. The fibroblast treatment visibly accelerated chronic wound closure compared with an FDA approved DFU treatment.

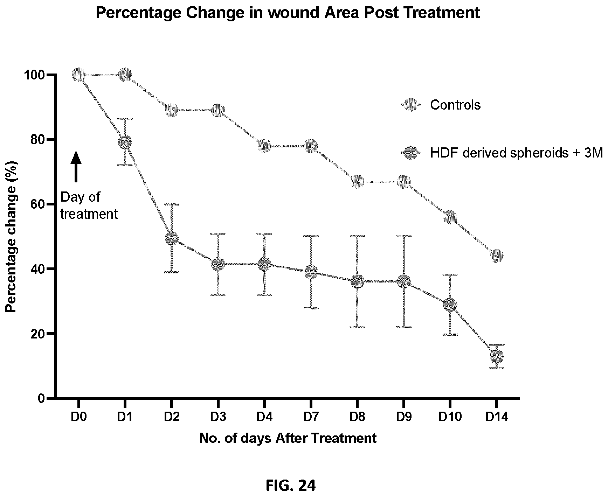

Fig. 24 is a graph of the percentage change in wound area post treatment. The control data is the higher of the two. The graph shows the percentage change over days using a control compared to HDF derived spheroids plus 3M Nexcare^{™}. The graph illustrates that that HDF increases the efficacy of 3M Nexcare^{™}. The spheroids significantly accelerated wound closure.

The results of this study indicated that human dermal spheroid fibroblasts healed the wound significantly faster than the control, mouse dermal fibroblast spheroids, and the Grafix^{®}. The wounds healed at day 5 with the human dermal fibroblast spheroids, at day 7 with the mouse dermal fibroblast spheroids, and at day 8 with Grafix^{®}. The human dermal fibroblast spheroids also controlled the inflammation significantly better than the other test materials by not seeing an increase in wound size after administration. In fact, there was an immediate reduction in wound size after administration of the human dermal fibroblasts. When comparing the human dermal fibroblasts spheroids to the mouse dermal fibroblast spheroid, the fibroblast spheroids healed the wound significantly faster.

### EXAMPLE 3

### BIOMARKER ANALYSIS OF SERUM AND WOUND TISSUE

IFN- γ is the master regulator of immune response and inflammation. IFN-γ is required for the repair of skin wounds in the proliferation phase due to its regulation of neutrophilic inflammatory responses. EGFR plays a crucial role in initiating the signaling that directs the behavior of epithelial cells. VEGF levels can influence the rate of wound closure/re-epithelialization, angiogenesis, granulation tissue formation, and the strength of the healed wound during the proliferative phase. IL-10 activated the STAT3 pathway that promotes vascularization to enable faster wound closure and attenuated scarring. The regenerative effects of IL-10 on the epithelium may be mediated through an interaction between fibroblasts and keratinocytes. Fibroblasts respond readily to IL-10 and have been found to have superior migration and invasion through provisional matrices to induce repair of wounds. IL-1 α is a proinflammatory marker that stimulates the activity of genes involved in inflammation. It reestablishes a pro-healing microenvironment characterized by lower levels of pro-inflammatory cells, cytokines and senescent fibroblasts, and higher levels of anti-inflammatory cytokines and growth factors. IL-6 induces the prelease of pro-inflammatory cytokines and chemokines into the wounds and is responsible for creating a reparative environment in wounds. Our results demonstrate that there is a significant increase in the IL-6 levels in the HDF group on day 7 in the serum showing that wound repair mechanism is carried out effectively in the treatment groups as compared to the controls.

These biomarkers can be used to quantitate the repair of skin wounds during a given treatment.

### Materials and Methods

For carrying out the biomarker assessment of the serum and skin samples collected in the experiment, a product from Millipore Sigma called MILLIPLEX^{®} Mouse Cytokine/Chemokine Magnetic Bead Panel was used in conjunction with a Luminex^{®} xMAP^{®} system according to the manufacturer protocol. The raw data collected was then analyzed, and statistical analysis carried out using the Graphpad Prism^{®} software.

### Results

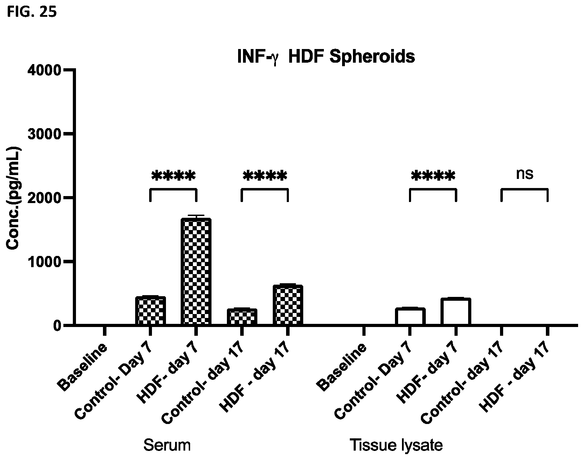

Fig. 25 illustrates INF-γ levels at days 7 and 17 in skin tissue and serum that have been treated with HDF spheroids or a control. The graph in Fig. 6 shows the concentration of INF-γ in the serum and the tissue lysate for control treatment and treatment with human dermal fibroblasts. These results demonstrate that there is a significant increase in the IFN-gamma levels in the HDF group on day 7 showing that wound repair mechanism is carried out effectively in the treatment groups as compared to the controls.

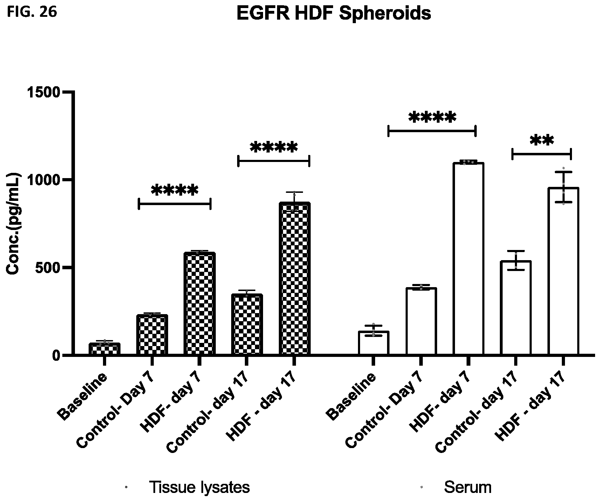

Fig. 26 illustrates EGFR levels at days 7 and 17 in skin tissue and serum that have been treated with HDF spheroids or a control. The graph shows the concentration of EGFR in the serum and tissue lysate for control treatment and treatment with human dermal fibroblasts. These results show an increased epithelialization in the HDF treatment group as compared to their controls on day 7 and 17 as the wound is healing. This indicates that EGFR stimulates cellular events critical to wound healing such as, keratinocyte proliferation, re-epithelialization, inflammation, and angiogenesis.

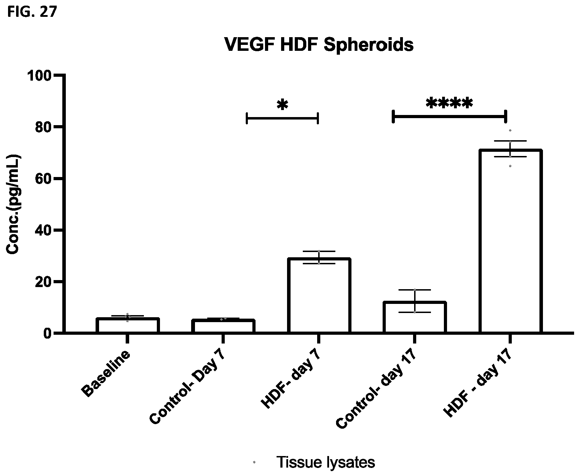

Fig. 27 shows VEGF levels at days 7 and 17 in skin tissue following treatment with human dermal fibroblasts or a control. The graph shows the concentration of VEGF for control treatment and HDF treatment. These results show a significant increase in the VEGF levels at day 7 in the HDF treatment group as compared to the controls. The levels are much higher (7 folds increased) on day 17 as compared to day 7 as the wound is healing and vascular tissue is being formed.

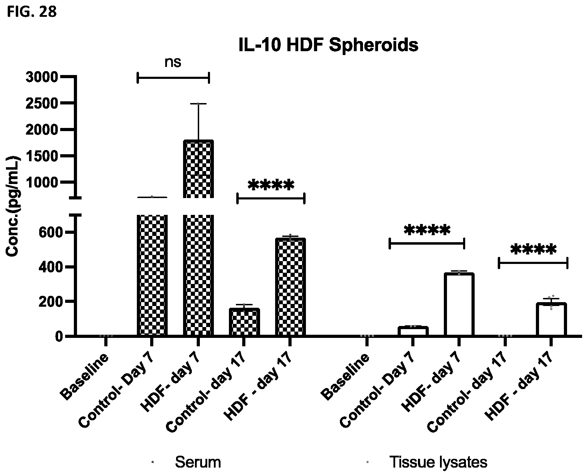

Fig. 28 shows IL-10 levels at days 7 and 17 in skin tissue and serum following treatment with human dermal fibroblasts or a control. These results demonstrate that there is a significant increase in the IL-10 levels in the HDF group on day 7 in the tissue lysates showing that wound repair mechanism is carried out effectively in the treatment groups as compared to the controls. Once the wound is completely healed (day 17), the levels start to return to their normal levels hence the levels are much lower than that on day 7.

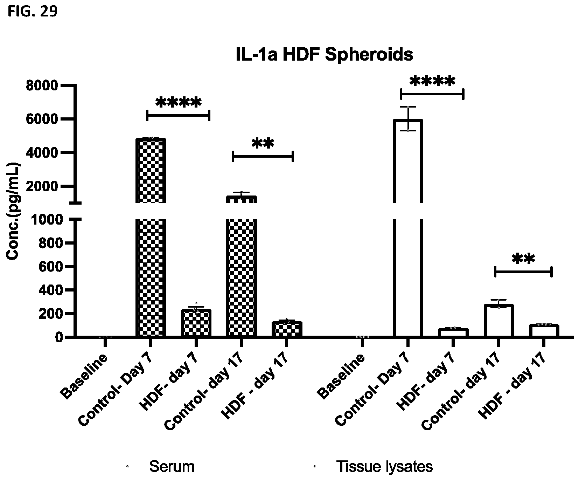

Fig. 29 shows IL-1a levels at days 7 and 17 in skin tissue and serum following treatment with human dermal fibroblasts or a control. The results demonstrate that there is a significant decrease in the IL-1a levels in the HDF group on day 7 showing that wound repair mechanism is carried out via the anti-inflammatory markers in the treatment groups as compared to the controls. On day 17, the levels are lesser than the levels on day 7 but still significantly lower.

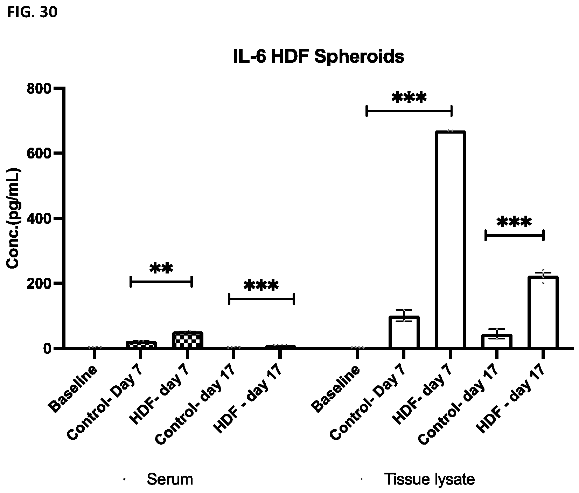

Fig. 30 shows IL-6 levels at days 7 and 17 in skin tissue and serum following treatment with human dermal fibroblasts or a control. The results demonstrate that there is a significant increase in the IL-6 levels in the HDF group on day 7 in the serum showing that wound repair mechanism is carried out effectively in the treatment groups as compared to the controls. In tissue lysates, HDF group shows a significant increase as compared to the control. Once the wound is completely healed (day 17), the levels start to return to their normal levels.

### References

1. Chuong, C.M., et al., What is the 'true' function of skin? Exp Dermatol, 2002. 11(2): p. 159-87.
2. Bainbridge, P., Wound healing and the role of fibroblasts. J Wound Care, 2013. 22(8): p. 407-8, 410-12.
3. Li, Z. and P. Maitz, Cell therapy for severe burn wound healing. Burns Trauma, 2018. 6: p. 13.
4. Kubo, K. and Y. Kuroyanagi, A study of cytokines released from fibroblasts in cultured dermal substitute. Artif Organs, 2005. 29(10): p. 845-9.
5. Spiekstra, S.W., et al., Wound-healing factors secreted by epidermal keratinocytes and dermal fibroblasts in skin substitutes. Wound Repair Regen, 2007. 15(5): p. 708-17.
6. https://www.ncbi.nlm.nih.gov/pmc/articles/PMC6888635/

References 1-6 listed above are incorporated by reference in full.

Although the present disclosure and its advantages have been described in detail, it should be understood that various changes, substitutions, and alterations can be made herein without departing from the spirit and scope of the invention as defined by the appended claims. Moreover, the scope of the present application is not intended to be limited to the particular embodiments of the process, machine, manufacture, composition of matter, means, methods, and steps described in the specification. As one of ordinary skill in the art will readily appreciate from the disclosure of the present disclosure, processes, machines, manufacture, compositions of matter, means, methods, or steps, presently existing or later to be developed that perform substantially the same function or achieve substantially the same result as the corresponding embodiments described herein may be utilized according to the present disclosure. Accordingly, the appended claims are intended to include within their scope such processes, machines, manufacture, compositions of matter, means, methods, or steps.

### ASPECTS OF THE INVENTION

1. An in vitro method of producing activated or un-activated single-cell or spheroid fibroblasts, exosomes from fibroblasts, lysate from fibroblasts, apoptotic bodies from fibroblasts, and/or fibroblast-related products or any combination thereof for introduction into and/or onto a substrate comprising one or more synthetic polymers and/or one or more biopolymers to generate a material to cover at least part of one wound and/or at least one burn of an individual.
2. The method of aspect 1, wherein the material is a fabric, scaffolding, or mesh.
3. The method of aspect 2, wherein the fabric is a skin-like fabric.
4. The method of any one of aspects 1-3, wherein the method is directly onto the patient.
5. The method of any one of aspects 1-4, wherein the single-cell or spheroid fibroblast, exosomes from fibroblasts, lysate from fibroblasts, apoptotic bodies from fibroblasts, and/or fibroblast-related products are autologous with respect to the individual.
6. The method of any one of aspects 1-4, wherein the single-cell or spheroid fibroblast, exosomes from fibroblasts, lysate from fibroblasts, or apoptotic bodies from fibroblasts and/or fibroblast-related products are allogeneic with respect to the individual.
7. The method of any one of aspects 1-4, wherein the single-cell or spheroid fibroblast, exosomes from fibroblasts, lysate from fibroblasts or apoptotic bodies from fibroblasts and/or fibroblast-related products cells are xenogeneic with respect to the individual.
8. A composition comprising:
   one or more biological components of (a) single-cell fibroblasts, spheroid fibroblasts, exosomes from fibroblasts, lysate from fibroblasts, apoptotic bodies from fibroblasts, conditioned media from fibroblasts, and/or fibroblast-related products; or any combination thereof and
   one or more substrate components of (b) synthetic polymer or biopolymer scaffolding, tray, plate, geometric formation, biologic fabric, mesh, polymer fabric or any combination thereof.
9. The composition of aspect 8, wherein component of (a) is separate from (b).
10. The composition of aspect 8, wherein component of (a) is comprised on and/or in (b).
11. The composition of any one of aspects 8-10, wherein the component of (b) comprises one or more apertures.
12. The composition of aspect 11, wherein the aperture was generated by perforation.
13. The composition of aspect 11 or 12, wherein the distribution of apertures in the substrate is random.
14. The composition of aspect 11 or 12, wherein the distribution of apertures in the substrate is patterned.
15. A method of treating one or more wounds and/or burns in an individual, comprising the step of administering an effective amount of the composition of any one of aspects 8-14 onto and/or adjacent to a wound or burn of the individual.
16. The method of aspect 15, wherein prior to the administering step, single cells and/or spheroids are applied to the substrate.
17. The method of aspect 16, wherein single cells and/or spheroids are applied with a device to the substrate.
18. The method of any one of aspects 15-17, wherein the substrate is a scaffolding comprising apertures of sufficient size to allow the migration of cells and/or cell product to the wound and/or burn.
19. The method of any one of aspects 15-18, wherein the substrate is a scaffolding comprising apertures of sufficient size to allow the securing of one or more spheroids in one or more apertures of the substrate.
20. The method of any one of aspects 15-17, wherein prior to the administering step, single cells and/or spheroids are applied in a gel-based biopolymer to the substrate.
21. The method of any one of aspects 15-18, wherein the substrate is a scaffolding comprising apertures of sufficient size to allow the migration of cells and/or cell product to the wound and/or burn.
22. The method of any one of aspects 15-18, wherein the substrate is a scaffolding comprising apertures of sufficient size to allow the securing of one or more spheroids in one or more apertures of the substrate.
23. The method of any one of aspects 15-17, wherein prior to the administering step single cells and/or spheroids and/or cell products are already present in a predetermined arrangement of apertures of the scaffolding, and then placed on the wound or burn.
24. The method of any one of aspects 18-23, wherein the arrangement of the apertures in the substrate is random.
25. The method of aspect 26, wherein the arrangement of the apertures in the substrate is patterned.
26. The method of aspect 25, wherein the arrangement of apertures in the substrate is patterned in a waffle pattern.
27. The method of any one of aspects 15-26, wherein the wound is a diabetic ulcer, bedsore, cut, surgical excision, surgical incision, amputation, biopsy, abrasion, infection site, or animal bite.
28. The method of any one of aspects 15-27, wherein the wound is internal to the individual.
29. The method of aspect 28, wherein at least part of the substrate component is resorbable.
30. The method of any one of aspects 15-27, wherein the wound is external to the individual.
31. The method of any one of aspects 15-30, wherein at least part of the composition is produced by a 3-D printer.
32. A method of producing the composition of any one of aspects 8-14, comprising the steps of:
   providing one or more components of (a) selected from the group consisting of single-cell fibroblasts, spheroid fibroblasts, exosomes from fibroblasts, lysate from fibroblasts, apoptotic bodies from fibroblasts, conditioned media from fibroblasts, fibroblast-related products, and a combination thereof;
   providing one or more components of (b) selected from the group consisting of synthetic polymer or biopolymer scaffolding, tray, geometric formation, biologic fabric, mesh, polymer fabric, and a combination thereof; and
   combining an effective amount of one or more components of (a) with one or more components of (b).
33. The method of aspect 32, wherein the providing of one or more components of (a) comprises three-dimensional (3D) printing of the one or more components of (a) onto the one or more components of (b).
34. The method of aspect 32, wherein the providing of one or more components of (b) comprises 3D printing the one or more components of (b).
35. The method of any one of aspects 32-34, wherein single cell and/or spheroids and/or cell products are applied with a device to a scaffolding with perforations to allow the migration of cells and cell product to the wound.
36. The method of any one of aspects 32-34, wherein single cell and/or spheroids and/or cell products are applied in a gel-based biopolymer to a scaffolding with perforations to allow the migration of cells and cell product to the wound.
37. The method of any one of aspects 32-34, wherein single cell and/or spheroids and/or cell products are applied already deposited in a predetermined arrangement of perforations of the scaffolding, and then placed on a wound and/or burn.
38. A kit comprising the composition of any one of aspects 8-14.
39. The kit of aspect 1, wherein the composition comprises a tray, plate, or mesh comprising one or more spheroids and/or cells thereon.

## Claims

1. An in vitro method of producing activated or un-activated single-cell or spheroid fibroblasts, exosomes from fibroblasts, lysate from fibroblasts, apoptotic bodies from fibroblasts, and/or fibroblast-related products or any combination thereof for introduction into and/or onto a substrate comprising one or more synthetic polymers and/or one or more biopolymers to generate a material to cover at least part of one wound and/or at least one burn of an individual.

2. The method of claim 1, wherein:
(a) the material is a fabric, scaffolding, or mesh, optionally wherein the fabric is a skin-like fabric; and/or
(b) the single-cell or spheroid fibroblast, exosomes from fibroblasts, lysate from fibroblasts, apoptotic bodies from fibroblasts, and/or fibroblast-related products are autologous with respect to the individual, or allogeneic with respect to the individual, or cells are xenogeneic with respect to the individual.

3. A composition comprising:
one or more biological components of (a) single-cell fibroblasts, spheroid fibroblasts, exosomes from fibroblasts, lysate from fibroblasts, apoptotic bodies from fibroblasts, conditioned media from fibroblasts, and/or fibroblast-related products; or any combination thereof; and
one or more substrate components of (b) synthetic polymer or biopolymer scaffolding, tray, plate, geometric formation, biologic fabric, mesh, polymer fabric or any combination thereof.

4. The composition of claim 3, wherein:
(i) component of (a) is separate from (b); or
(ii) component of (a) is comprised on and/or in (b).

5. The composition of claims 3 or 4, wherein the component of (b) comprises one or more apertures.

6. The composition of claim 5, wherein:
(a) the aperture was generated by perforation; and/or
(b) the distribution of apertures in the substrate is random, or patterned.

7. The composition of any one of claims 3-6 for use in a method of treating one or more wounds and/or burns in an individual, comprising the step of administering an effective amount of said composition onto and/or adjacent to a wound or burn of the individual, optionally wherein prior to the administering step, single cells and/or spheroids are applied to the substrate, further optionally wherein single cells and/or spheroids are applied with a device to the substrate.

8. The composition for use according to claim 7, wherein:
(A)
(i) the substrate is a scaffolding comprising apertures of sufficient size to allow the migration of cells and/or cell product to the wound and/or burn; and/or
(ii) the substrate is a scaffolding comprising apertures of sufficient size to allow the securing of one or more spheroids in one or more apertures of the substrate; and/or
(B)
(i) prior to the administering step, single cells and/or spheroids are applied in a gel-based biopolymer to the substrate; and/or
(ii) the substrate is a scaffolding comprising apertures of sufficient size to allow the migration of cells and/or cell product to the wound and/or burn, or the securing of one or more spheroids in one or more apertures of the substrate; and/or
(C) prior to the administering step single cells and/or spheroids and/or cell products are already present in a predetermined arrangement of apertures of the scaffolding, and then placed on the wound or burn.

9. The composition for use according to claim 8, wherein the arrangement of the apertures in the substrate is random, optionally wherein the arrangement of the apertures in the substrate is patterned, further optionally wherein the arrangement of apertures in the substrate is patterned in a waffle pattern.

10. The composition for use according to any one of claims 7-9, wherein:
(a) the wound is a diabetic ulcer, bedsore, cut, surgical excision, surgical incision, amputation, biopsy, abrasion, infection site, or animal bite; and/or
(b) the wound is internal to the individual, optionally wherein at least part of the substrate component is resorbable, or the wound is external to the individual; and/or
(c) at least part of the composition is produced by a 3-D printer.

11. A method of producing the composition of any one of claims 3-6, comprising the steps of:
providing one or more components of (a) selected from the group consisting of single-cell fibroblasts, spheroid fibroblasts, exosomes from fibroblasts, lysate from fibroblasts, apoptotic bodies from fibroblasts, conditioned media from fibroblasts, fibroblast-related products, and a combination thereof;
providing one or more components of (b) selected from the group consisting of synthetic polymer or biopolymer scaffolding, tray, geometric formation, biologic fabric, mesh, polymer fabric, and a combination thereof; and
combining an effective amount of one or more components of (a) with one or more components of (b).

12. The method of claim 11, wherein:
(i) the providing of one or more components of (a) comprises three-dimensional (3D) printing of the one or more components of (a) onto the one or more components of (b); or
(ii) the providing of one or more components of (b) comprises 3D printing the one or more components of (b).

13. The method of claims 11 or 12, wherein:
(a) single cell and/or spheroids and/or cell products are applied with a device to a scaffolding with perforations to allow the migration of cells and cell product to the wound; or
(b) single cell and/or spheroids and/or cell products are applied in a gel-based biopolymer to a scaffolding with perforations to allow the migration of cells and cell product to the wound; or
(c) single cell and/or spheroids and/or cell products are applied already deposited in a predetermined arrangement of perforations of the scaffolding, and then placed on a wound and/or burn.

14. A kit comprising the composition of any one of claims 3-6.

15. The kit of claim 14, wherein the composition comprises a tray, plate, or mesh comprising one or more spheroids and/or cells thereon.
